# EUROPEAN PATENT SPECIFICATION

(11) **EP 1 141 021 B1**
(45) Date of publication and mention of the grant of the patent: **08.09.2004**
(21) Application number: 99968357.6
(22) Date of filing: 20.12.1999
(51) Int. Cl.: C07K 14/765

(54) **REMOVAL/PURIFICATION OF SERUM ALBUMINS**
ENTFERNUNG ODER REINIGUNG VON SERUMALBUMIN
ELIMINATION/PURIFICATION D'ALBUMINES SERIQUES

(30) Priority: 22.12.1998 SE 9804465
(43) Date of publication of application: 10.10.2001
(73) Proprietor: Amersham Biosciences AB, 751 84 Uppsala (SE)
(72) Inventor: REGBERG, Tor, S-114 41 Stockholm (SE); ELLSTRÖM, Christel, S-754 33 Uppsala (SE)
(74) Representative: Rollins, Anthony John
(86) International application number: PCT/EP1999/010123
(87) International publication number: WO 2000/037501

(56) References cited:
- WO-A-96/02573
- WO-A-98/08603
- WO-A-99/33860

## Description

### Technical field

The present invention concerns a method for the selective separation/removal of a mammalian serum albumin from a solution containing a mixture of proteins in order to obtain a solution/preparation that is substantially devoid of one or more of the other proteins, for instance immunoglobulins and/or serum albumin of species origin other than the separated/removed serum albumin.

### Technical background

For a long time there has been a large demand for purified mammalian serum albumins, for instance serum albumin of human or bovine origin (HSA and BSA, respectively). For HSA this has mainly depended on its therapeutic use as a plasma volume expander. Originally serum albumins were obtained from sera/plasma of the appropriate species origin. For some years the focus has been to produce serum albumins recombinantly, in particular HSA. For bacterially produced recombinant forms, it has become urgent to remove host cell contaminants because they may be hazardous in vivo to mammals. It has become apparent that preparing HSA in transgeneic animals should be benificial, for instance in transgeneic cows. This latter alternative, however, has the drawback that HSA will be present in mixture with the HSA analogue of the host animal (for instance with BSA if HSA is produced in cows). This has created novel purification problems, for instance the specific removal of BSA from HSA.

Various forms of affinity binding including ion exchange binding have earlier been applied to the purification of serum albumins. For affinity binding the general goal has been to find chromatographic media (ligand attached to a chromatographic base matrix) that provide sufficient specificity in order to remove either predetermined contaminants or the serum albumin desired as the end product from complex mixtures. The term "binding" includes chromatographic as well as batch-wise alternatives.

Illustrative examples of ligands previously used and having selectivity for serum albumins are given by Theodore Peters in All about Albumin - Biochemistry, Genetics and Medical Applications, (Ed. Theodore Peters, Jr., Academic Press (1996) pages 77-126. Among low molecular weight ligands there may be mentioned certain dyes of rather complex structure, for instance Cibacron Blue.

WO 9602573 discloses a method for selectively purifying human serum albumin from whole milk or whey by a process comprising an affinity chromatographic step utilizing triazine dye molecules as affinity ligands.

WO 9808603 discloses a number of aromatic compounds that can be used as general protein binders under the appropiate conditions. The compounds includes 2-mercapto-benzothiazoles among several others. Serum albumin is used as one of many model compounds.

The methods concerned in the present invention generally provide that a mixture of compounds, in particular proteins, containing a serum albumin is contacted with a ligand that has affinity for the serum albumin and that is attached to a base matrix. The conditions are adjusted such that the serum albumin becomes selectively bound (adsorbed) to the ligand. The matrix-bound affinity ligand may be represented by the formula I

M-B-X,

where M is the matrix, X the affinity ligand (ligand structure) and B a spacer group through which X is attached to the matrix. M may contain several groups X linked through spacers that may be equal or unequal to B.

The expression "a ligand that is attached to a matrix" includes also ligands that are possible to attach to a matrix after having bound a serum albumin.

### Objectives of the invention

The first objective of the invention is to provide novel affinity methods for the selective removal of a serum albumin from a mixture of proteins in order to produce the serum albumin in substantially pure form or a preparation essentially free of the removed serum albumin.

A second objective is to provide an affinity method as defined above which is selective for a serum albumin that exist in mixture with one or more other serum albumins.

### The invention

It has now been discovered that there are compounds that selectively bind albumin to be found among bicyclic compounds in which a five-membered heterocycle is fused with a benzene ring. The heterocycles concerned contain two or three heteroatoms selected from oxygen, nitrogen and sulphur. It has also been found that this type of albumin-binding compounds can be used as affinity ligands attached to matrices for the purification/removal of serum albumins from liquids. It has also been found that this ability to bind albumin may differ depending on the species origin of the serum albumin.

The inventive method thus has the characterizing feature that the ligand X defined above has been selected from the group of compounds defined in the preceding paragraph and linked to an affinity matrix M through a spacer B. In particular X complies with the structure according-to formula II: 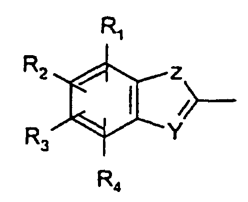 The free valence binds to the spacer B.

R₁, R₂ R₃ and R₄ are selected from hydrogen, electron-withdrawing groups and lower alkyl groups (C₁₋₁₀) wherein at least one of R₁₋₄ exhibits an electron withdrawing group. Particular important alkyl groups (R₁₋₄) contain 1-3, preferably one, carbon atom. Particular important electron withdrawing groups are halogens, such as F, Cl and Br, which also may enhance a hydrophilic character of the ligand. A well known hydrophilic group is hydroxy (HO-) that, like the halogens, when present on an alkyl group will enhance the hydrophilic character of the latter. Particular interesting groups R₁₋₄ comprise C₁₋₁₀, such as C₁₋₄, alkyl groups substituted with 1-3 halogen atoms, preferably fluorine, on carbons at none, one or two atoms distance from the ring structure. One, two or three of R₁₋₄ may be different from hydrogen, although it is believed that in the preferred ligands X one or two of R₁₋₄ should not be hydrogen. Non-hydrogen groups R₁₋₄ may be located in the 4-, 5-, 6- and/or 7-position with preference for positions 4 and/or 6.

Z and Y are heteroatoms selected from nitrogen, oxygen and sulphur, possibly carrying one or two organic groups or hydrogens in addition to the heterocyclic ring they are part of. Normal valence rulesfor stable compounds apply, i.e. the nitrogen may carry one or two, the sulphur one and the oxygen none additional group(s). The organic groups may be selected according to the same principles as R₁₋₄, with preference for smaller alkyl groups and hydrogen. In the heterocyclic ring a nitrogen atom and a sulphur atom can be positively charged if they bind to other atoms or groups via four and three valencies, respectively.

In the preferred ligand structures X, Z and Y are NR'R'' and NR''', respectively. R', R" and R''' are selected according to the same principle as R₁₋₄ or is a free electron pair, with the proviso that a selected R₁₋₄ group should provide a saturated sp³-hybridised carbon atom binding directly to the heterocyclic ring. The preferred ligand structures of this embodiment comprise that at most one of R' and R" is a free electron pair or a hydrogen. In this latter embodiment R''' is typically a free electron pair.

The general idea is that the useful types of ligands shall have a pronounced hydrophilicity in order to increase the desired selectivity for serum albumins and to minimize binding of other kinds of proteins via hydrophobic interactions. It is therefore believed that ligand structures that selectively bind albumin will be found by selecting or screening among water-soluble organic compounds that
a) comprise a benzene ring fused to a five-membered heterocycle containing two or three, preferably two, heteroatoms selected from nitrogen, oxygen and sulphur and
b) have been attached to a matrix M via a spacer B replacing a group or atom at any of the positions 1, 2, 3, 4, 5, 6 and 7
for selective binding to serum albumin. Once a compound according to formula II has been pretested or prescreened and found capable of binding selectively a serum albumin, this finding may be used by anyone to selectively remove the serum albumin as discussed above.

Particularly interesting compounds have two ring heteroatoms which are present in position 1 and 3, respectively, such as those compounds complying with the general structure given in formula III. Attachment to a matrix is preferably at the 2-position of the ring system. The screening methodology may be of the same kind as given in the Experimental Part. 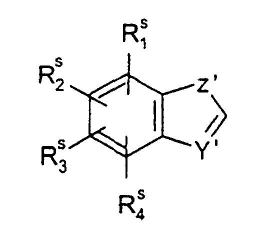 Z' and Y' are heteroatoms selected according to the same rules as for Z and Y. In case they comprise nitrogen as the heteroatom they may be NRₛ'Rₛ'' and NRₛ''', respectively. The substituents R^{s}₁₋₄ and Rₛ', Rₛ'' and Rₛ''' and the groups/atoms Y' and Z' are selected such that the compound III is soluble in water at a pH-value in the interval 1-11, preferably 1-7 (25°C). By solubility in water is meant at least 0.01 mg/ml, with preference for at least 0.1 mg/ml. Typically this means that, when one or more of R^{s}₁₋₄ (for instance as defined for R₁₋₄ above) are alkyl groups with a relatively long carbon chain, there should also be present one or more hydrophilic substituents, for instance hydroxy, amino, carboxy and other related substituents (ether, thioether, amido, ester etc), on the chain. The chain may also be broken by one or more heteroatoms, such as sulphur (thioether), oxygen (ether) or nitrogen atoms (secondary, tertiary or quaternary amino). Preferably the compound (and the ligand structure) should exhibit a charge (positive or negative) in a part of this pH interval (positive in the lower part and/or negative in the upper part or positive all throughout the pH-interval 1-10. It is believed that screening for binding preferentially should take place at a pH
where the ligand structure have a charge (≥ 0, preferably > 0. This includes that the charge may be located at a nitrogen in any of the groups R^{s}₁₋₄ and Rₛ', Rₛ'' and Rₛ''' or even in the spacer B.

At the priority the preferences with respect to R^{s}₁₋₄ and Rₛ', Rₛ'' and Rₛ''' were the same as for R^{s}₁₋₄, R', R'' and R''' and Y and Z.

The hydrogen (H) in position 2 may be replaced by the part of the spacer B that is next to X in formula I, for instance a part containing a spacer chain of at most 10 atoms in length.

### The matrix and the attachment of the ligand to the matrix.

In the preferred modes of the invention the ligand is attached to a base matrix that is insoluble in the aqueous media used. Such matrices often are based on polymers that expose a hydrophilic surface to the aqueous media used, i.e. expose hydroxy (-OH), carboxy (-COOH), carboxamido (-CONH₂, possibly in N- substituted forms), amino (-NH₂), oligo- or polyethylenoxy groups on their external and, if present, also on internal surfaces. Typically the matrices are of the same kind as those normally used as chromatographic matrices. The polymers may, for instance, be based on polysaccharides, such as dextran, starch, cellulose, pullulan, agarose etc, which if necessary have been crosslinked, for instance with bisepoxides, epihalohydrins, 1,2,3-trihalo substituted lower hydrocarbons, to provide a suitable porosity and rigidity. The matrices may also be based on synthetic polymers, such as polyvinyl alcohol, poly hydroxyalkyl acrylates, poly hydroxyalkyl methacrylates, poly acrylamides, polymethacrylamides etc. In case of hydrophobic polymers, such as those based on divinyl and monovinyl substituted benzenes, the surfaces of the matrices are often hydrophilized to expose hydrophilic groups as defined above.

The matrices may also be of inorganic nature, e.g. silica, zirkonium oxide etc.

Physically the insoluble matrices may be in the form of porous monoliths or in beaded/particle form that can be either porous or non-porous. Matrices in beaded/particle form can be used either as a packed bed or in suspended form. Suspended forms include so called classified expanded beds and pure suspensions in which the particles/beads are moving round completely. In case of monoliths, packed bed and classified expanded beds, the separation procedure may be classified as a normal chromatography with a concentration gradient of adsorbed molecules being established along the flow direction. In case of pure suspension the separation procedure will be in the batch wise mode.

For suspensions, the beads/particles may contain a densifying filler material that will permit higher flow rates in case of classified expanded beds and facilitate sedimentation of the beads/particles after adsorption. See for instance WO-A-9717132 (Amersham Pharmacia Biotech AB) and WO-A-9200799 (Upfront Chromatography).

The matrices may also comprise so called extenders carrying several groups -B-X. Extenders are often polymeric providing attachments of several ligands per spacer. Extenders are often hydrophilic and called tentacles etc. See for instance International Patent Application PCT/SE98/00189 (Amersham Pharmacia Biotech AB).

A spacer is typically introduced to improve the availability of the ligand and/or facilitate the chemical coupling of the ligand to the matrix. Spacers often comprise a hydrocarbon chain that has a length between 1-50 atoms. The hydrocarbon chain may be straight, branched or cyclic and optionally broken with one or more ether oxygen or amino nitrogen atoms and/or optionally substituted with one or more hydroxy, lower alkoxy, or amino group (-NH₂/NH₃⁺, where each hydrogen may be replaced with a lower alkyl or a lower acyl group). By lower alkyl or lower acyl group is primarily intended C₁₋₁₀ alkyls/acyls. The spacer group may also, depending to coupling methodology, comprise ester, amido, thioether, etc groups that have the sufficient hydrolytic stability. This latter groups may be present in the spacer either alone or combined with each other and/or with the appropriate hydrocarbon chain(s).

In the spacer the atom binding directly to the ligand structure X should preferably be a sulphur atom, preferably bound to a saturated carbon atom in the spacer. In other alternatives of the spacer, the atom next to the ligand structure X may be an oxygen, a nitrogen (amino or amido nitrogen), a carbon (for instance a saturated carbon, carbonyl carbon etc). Typical saturated carbon atoms are only binding carbons and/or hydrogen.

The spacer, in particular the part closest to ligand structure X, may influence the ability to bind to serum albumin. The discussion above therefore primarily indicates the group of spacers that is to be screened for finding the optimal spacer for various ligand structures.

It can be envisaged that the ligand potentially also will be attached to the matrix by methods involving non-covalent bonding, such as physical adsorption or biospecific adsorption. In these cases the ligand may be covalently attached, via a spacer structure, to some type of carrier molecule that provides the ligand with sufficient adsorption ability relative adsorbing structures in the matrix. The carrier molecule and the adsorbing structures are in these cases regarded as being part of the matrix (M'). Pairs of carrier molecules and adsorbing structures may be represented by the biotin-strepavidin system.

As a potential alternative, the ligand may be in soluble form that subsequent to binding to a serum albumin is insolubilized. This may be accomplished, for instance, by having the ligand conjugated to biotin and insolubilizing by contacting the formed complex between the serum albumin and the soluble ligand-biotin conjugate with a strepavidin-matrix. The spacer in this mode will be the grouping linking the ligand to biotin.

### The sample containing the serum albumin to be removed/purified

The serum albumin to be removed and/or purified typically exists in mixture with other proteins and/or biomolecules. Illustrative examples are blood preparations (such as plasma and serum), fermentation liquids obtained from cultured host cells that have been transformed to express a serum albumin, biological fluids obtained from transgeneic mammals transformed to produce a serum albumin of another species, and also various working up preparations derived from any one of these types of liquids. Important biological fluids from transgeneic mammals are milk and milk fractions such as whey. In case of liquids derived from transgeneic animals the liquids will often contain also the normal serum albumin of the species concerned.

### Procedural steps for binding

During the adsorption step the conditions are selected so as to promote binding between the ligand and the serum albumin intended. At the same time the conditions are selected such that binding between the ligand and other proteins present is surpressed, for instance hydrophobic interactions.

For pH this means that the pH-value shall be selected above the value at which serum albumin-unfold. This value in turn may depend on ionic strength and temperature. The uppermost end of the pH is limited at the pH value at which hydrolytic degradation becomes significant. As a general rule of thumb, the appropriate pH values will therefore be found in the range 4-10. However, our present findings suggest that the binding may require a positive charge on the ligand structure. The very broad range 4-10 may therefore only be applicable to ligand structures, including the spacer B, carrying a pH-independent charge, for instance Z being a quaternary ammonium group. In case one or more of Z, Y, R^{s}₁₋₄ and Rₛ', Rₛ'' and Rₛ''' or B comprise a nitrogen that can be protonated and deprotonated in water, the ligand structure X plus the spacer B may exhibit a positive charge which is pH-dependent. The lower part of the pH interval 4-10 will then be applicable, for instance pH 4-7 or even lower such as 4-6.

The ionic strength should be selected in the interval correponding to 0-3 M NaCl. Ionic strengths at the upper part of this interval are likely to promote binding via hydrophobic interactions. This means that albumin binding will be promoted at higher ionic strengths, but simultaneously also proteins with pronounced hydrophobic regions will bind more efficiently thereby decreasing the selectivity for serum albumins. The optimal ionic strength will therefore, among others, depend on what is to removed from what. The temperature is typically selected in the interval 0-40°C with preference for 4-37°C.
The optimal values of pH, temperature and ionic strength will depend on the species origin of the serum albumin to be removed/purified, contaminants, the ligand structure attached to the matrix etc.

After adsorption, the serum albumin may be further worked up, for instance by first desorbing the serum albumin and subsequently subjecting it to further adsorption steps, for instance on a cation or anion exchanger, an hydrophilic matrix exhibiting hydrophobic groups (HIC-media) etc. Suitably desorption conditions may include change of pH, of ionic strength, of temperature and/or addition of compounds interfering with binding. Compounds that interfere with the binding may mimick the ligand structure X present on the matrix. It may, for instance, be a compound according to formula III possibly also exibiting a relevant the part of the spacer B. The main rules should then of course be not to change the conditions so that the serum albumin becomes irreversibly denatured. The adsorption step may be preceded by other steps, which for instance may be based on ion exchange or immune ligands. Illustrative examples of steps that can be combined with the present inventive adsorption step are given in US 5440018; US 5521287 and EP 699687 (all in the name of Green Cross Corp).

The goal of the full separation processes is to obtain serum albumin preparations intended for use in vivo. This means that the preparation should contain < 0,01 %, such as < 0,001 % proteins that are heterologous to the species to which the preparation is to be administered. HSA preparations, for instance, that are to be used in humans shall contain < 0,01 %, such as < 0,001 % BSA. All percentages are w/w. This means that in order to reach a therapeutically acceptable purity the serum albumin preparations obtained directly from the adsorption step of the present invention often will need to be combined with further working up steps, either prior or subsequent to the inventive adsorption step.

The invention is further defined in the appended claims and will now be further described by experimental support regarding the most preferred fragment.

### EXPERIMENTAL PART

The purpose of the study was to immobilize a number of selected heterocycles condensed to a benzene ring (benzo compounds) to an epoxy activated matrix (Sepharose 4FF, Amersham Pharmacia Biotech AB, Uppsala, Sweden) and test them for binding to human serum albumins and IgG in PBS buffer at pH 7. In order to facilitate attachment to the matrix the compounds were selected to carry a thiol group (at the 2-position). The ligand structures were:

| No | Ligand structure X | Manufacturer of coupled compound | Subst. (mole/ ml gel | Vt ml |
|---|---|---|---|---|
| 1 | Benzimidazol-2-yl | Aldrich | 15.1 | 1.2 |
| 2 | 5-methyl-benzimidazol-2-yl | Aldrich | 14.4 | 1.2 |
| 3 | Benzothiazol-2-yl | Aldrich | 8.4 | |
| 4 | Benzoxazol-2-yl | Aldrich | 12.2 | 1.2 |
| 5 | 4-bromo-6-(trifluoromethyl)-benzimidazol-2-yl | Maybridge | 15.1 | 1.2 |
| 6 | 6-ethoxy-benzothiazol-2-yl | Aldrich | 9.6 | 1.1 |
| 7 | 5-nitro-benzimidazol-2-yl | Aldrich | 15.1 | 1.2 |
| 8 | 6-nitro-benzothiazol-2-yl | TCI-GB | 6.8 | 1.2 |
| 9 | 5-chloro-benzothiazol-2-yl | Aldrich | 9.6 | 1.2 |
| 10 | 5-methoxy-benzimidazol-2-yl | Aldrich | 14.7 | 1.1 |
| 11 | 4-chloro-6-(trifluoromethyl)-benzimidazol-2-yl | Maybridge | 14.3 | 1.2 |
| 12 | 5-chloro-1-isopropyl-benzimidazol-2-yl | Maybridge | 18.2 | 1.2 |
| 13 | 5,6-dichloro-benzimidazol-2-yl | Maybridge | 15.1 | 1.2 |
| 14 | 5-chloro-6-fluoro-benzimidazol-2-yl | Maybridge | 4.9 | 1.1 |

**Test proteins:** Human Serum Albumin (HSA) (Sigma), Bovine Serum Albumin (BSA) (Sigma) and IgG human normal (Pharmacia AB = Pharmacia & Upjohn, Stockholm, Sweden).

### Buffers used in binding experiments:

1. PBS pH 7
2. 20 mM citrate buffer. Dissolve 2.52 g citric acid monohydrate and 1.90 g citric acid monosodium salt in 1000 ml MilliQ water.
3. 2 M NaCl. Dissolve 116.88 g in 1000 ml MilliQ.
4. West. Add 6.8 g sodium acetate to 840 ml MilliQ water. Adjust pH with hydrochloric acid. Add 47.9 lactose, 0.44 g calcium chloride and 20 ml 2M NaCl. Add MilliQ water to 1000 ml.

### Test solutions:

HSA (2 mg/ml in PBS)
HSA (2 mg/ml in West)
BSA (2 mg/ml in West)
IgG (1.5 mg/ml in PBS; dilute 1 ampoule (165 mg/ml) to 100 ml)
**Synthesis of affinity media:** 100 ml of drained gel (Sepharose FF (Amersham Pharmacia Biotech AB, Uppsala) were washed on a glass filter with 400 ml of distilled water and transferred to a round bottomed glass flask together with 5.5 g of sodium hydroxide dissolved in 30 ml of distilled water. The mixture was kept at 35°C. 80 ml of 1,4-bis(epoxypropoxy)-butan were added under vigourous stirring. After modest stirring for two hours the gel was washed with 1.5 1 of distilled water.

In each synthesis 1 mmole of the compound to be coupled (compounds 1-15) was dissolved in 5 ml DMSO. The epoxy gel was washed with DMSO followed by addition of the ligand solution to 5.0 g of gel. 2 drops of 45% sodium hydroxide solution were added to each reaction flask (pH about 11). The reaction vessels were incubated at 40°C for 60 hours. The gels were filtered on glass funnels and were washed with the following solvents: 1. DMSO; 2. DMSO/water (1:1); 3. water; 4. THF; 5. water; 6. ethanol; and 7. water. The substitution degree was determined by elemental analysis.

**Packing of columns:** The gel slurry was poured into the column and packed by sucking the solution out with a syringe attached to the column outlet. The adapter was mounted on the column and the gel was equlibrated with about 10 columns of PBS buffer.

**Binding of HSA or human IgG in PBS pH 7 - elution with citrate pH 3:** The column was equilibrated with PBS pH 7 after which 2.000 ml HSA (2 mg/ml) or IgG (16.5 mg/ml) was applied. The column was eluted first with buffer of pH 7 and then with buffer of pH 3 (citrate; wash buffer).

**Binding of HSA and BSA in West pH 4.6 - step wise elution with PBS pH 7 and citrate pH 3 :** The column was equilibrated in West buffer pH 4.6 and 2.000 ml HSA (2 mg/ml) or BSA (2 mg/ml) was applied. Elution was made West pH 4.6 then with PBS pH 7 and finally with citrate pH 3.0 (wash).

### Results:

Binding of HSA or human IgG in PBS pH 7 - elution with citrate pH 3: Based on conventional ways of interpreting the chromatogram recorded, none of the ligand structures showed binding to IgG or HSA. The buffer was the standard for this type of experiments with serum albumin (PBS pH 7). In spite of these negative results the present inventors went further on and analysed in more detail the shape and position of the peaks of the chromatograms. It was found that for HSA the chromatograms sometimes looked somewhat different for different ligand structures. All chromatograms for IgG looked the same and the position of the eluted IgG suggested no interaction/binding. For seven (3, 4, 6, 8, 9, 12 and 13) out of the 14 tested ligand structures the chromatograms looked the same with the HSA peak located at the same elution volume and having the same shape.

The HSA chromatograms from the other seven columns (1, 2, 5, 7, 10, 11 and 14) could be paired, two by two, due to a similarity and/or retardation of the peaks compared to the peaks for the previously discussed ligand structures. The ligand structures 1 and 2 showed two peaks in the flow through and ligand structures 7 and 14 showed peaks with a shoulder. At least the latter result together with the retardation suggest an interaction with the media. Ligand structures 5 and 11 gave retarded peaks that were tailed. A very small elution peak and a small peak when reequilibrating the column could also be seen. The tailing and retardation suggested some type of weak interaction with the ligand structure on the media. Ligand structure 10 gave a small peak when reequilibrating the column.

Binding of HSA or BSA in West pH 4.6 - step wise elution with PBS pH 7 and citrate pH 3: The column with ligand structure 11 was studied with HSA and with BSA. The chromatograms indicated that all HSA applied and a part of the BSA applied were bound in West pH 4.6 (no HSA was eluted until PBS pH 7 was applied, one portion/peak of BSA eluted with West 4.6 and another with PBS pH 7).

## Claims

1. A method for selectively enriching/removing a serum albumin from a mixture of other compounds by contacting said mixture with a ligand (= X), said ligand
a) having affinity for and enabling binding of the serum albumin and
b) being attached via a spacer (= B) to a base matrix (= M') insoluble in the aqueous media used, the matrix with the attached ligand being represented by
M-B-X
where M is the matrix, B the spacer and X the affinity ligand, with the provision that M may contain further groups X linked via a spacer,
**characterized in that** said ligand X has been selected among serum albumin-binding structures complying with the formula 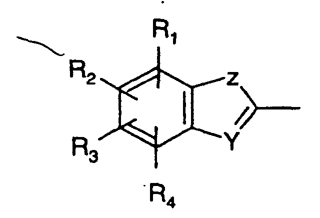 in which
a) the free valence bind to the spacer B;
b) R₁₋₄ are selected from hydrogen, electron-withdrawing groups, such as halogens and lower alkyl groups (C₁₋₁₀), wherein at least one of R₁₋₄ exhibits an electron withdrawing group, preferably selected among halogens;
c) Z and Y are selected among oxygen, sulphur or nitrogen, with the provision that the nitrogen may carry a positive charge.

2. The method according to claim 1, **characterized in that** contact between the mixture and the media M-B-X is done in an aqueous media having a pH at which the -B-X carries a positive charge.

3. The method according to any one of claims 1-2, **characterized in that** the spacer has a sulphur atom next to X.

4. The method according to any one of claims 1-3, **characterized in that** Z and Y are nitrogens, one of which binding to a hydrogen and the ligand structure being charged depending of pH.

5. The method of anyone of claims 1-4, **characterized in that** said mixture derives from a host in which said serum albumin is human serum albumin.

6. The method of anyone of claims 1-5, **characterized in that** said ligand is attached covalently to said matrix.

7. The method of anyone of claims 1-6, **characterized in that** after the adsorption step said serum albumin is eluted from said affinity adsorbent and if necessary further processed.

8. A method for screening for ligands structures that, when attached to an affinity matrix, selectively bind albumin, **characterized in that** water-soluble compounds that exhibit a benzene ring substituted with at least one electronwithdrawing group fused to a 5-membered heterocycle containing two or three heteroatoms, preferably two, selected from nitrogen, oxygen and sulphur after having been attached to a matrix, preferably in the 2-position, are screened for selective binding to albumin.

9. The method of claim 8, **characterized in that** screening is taking place in aqueous solutions at a pH at which the ligand stucture including the spacer binding to the matrix is positively charged.

## Patentansprüche

1. Verfahren zum selektiven Anreichern/Entfernen von Serumalbumin aus einer Mischung von anderen Verbindungen durch Inkontaktbringen der Mischung mit einem Liganden (= X), wobei der Ligand
a) eine Affinität gegenüber Serumalbumin aufweist und eine Bindung davon ermöglicht und
b) über einen Spacer (= B) an die Grundmatrix (= M'), welche in dem verwendeten wässrigen Medium unlöslich ist, gebunden ist, wobei die Matrix mit dem sich daran befindlichen Liganden durch
M-B-X
dargestellt wird,
worin M die Matrix ist, B der Spacer ist und X der Affinitätsligand ist, unter der Voraussetzung, dass M weitere Gruppen X, welche über einen Spacer gebunden sind, enthalten kann, **dadurch gekennzeichnet, dass** der Ligand X unter den Serumalbumin-bindenden Strukturen ausgewählt wurde, welche mit der Formel einhergehen 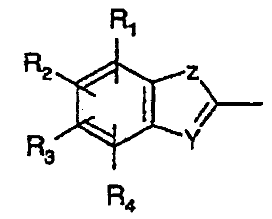 worin
a) die freie Valenz an den Spacer bindet;
b) R₁₋₄ aus Wasserstoff, elektronenziehenden Gruppen, wie Halogen, und Niederalkylgruppen (C₁₋₁₀), worin mindestens eine von R₁₋₄ eine elektronenziehende Gruppe bedeutet, vorzugsweise ausgewählt aus Halogenen, ausgewählt sind;
c) Z und Y ausgewählt sind aus Sauerstoff, Schwefel oder Stickstoff, unter der Voraussetzung, dass Stickstoff eine positive Ladung tragen kann.

2. Verfahren nach Anspruch 1, **dadurch gekennzeichnet, dass** der Kontakt zwischen der Mischung und dem Medium M-B-X in einem wässrigen Medium mit einem pH, bei dem -B-X eine positive Ladung trägt, erreicht wird.

3. Verfahren nach einem der Ansprüche 1 bis 2, **dadurch gekennzeichnet, dass** der Spacer ein Schwefelatom neben dem X aufweist.

4. Verfahren nach einem der Ansprüche 1 bis 3, **dadurch gekennzeichnet, dass** Z und Y Stickstoffatome, von denen eines an ein Wasserstoffatom bindet, und die Ligandstruktur in Abhängigkeit des pHs geladen ist.

5. Verfahren nach einem der Ansprüche 1 bis 4, **dadurch gekennzeichnet, dass** die Mischung sich von einem Wirt ableitet, worin das Serumalbumin Humanserumalbumin ist.

6. Verfahren nach einem der Ansprüche 1 bis 5, **dadurch gekennzeichnet, dass** der Ligand an die Matrix kovalent gebunden ist.

7. Verfahren nach einem der Ansprüche 1 bis 6, **dadurch gekennzeichnet, dass** nach dem Adsorptionsschritt das Serumalbumin aus dem Affinitätsadsorptionsmittel eluiert wird und, wenn dies notwendig ist, weiter verarbeitet wird.

8. Verfahren zum Screenen nach Ligandstrukturen, welche selektiv an Albumin binden, wenn sie an eine Affinitätsmatrix gebunden sind, **dadurch gekennzeichnet, dass** wasserlösliche Verbindungen, welche einen Benzolring aufweisen, der mit mindestens einer elektronenziehenden Gruppe substituiert ist und an einen 5-gliedrigen Heterocyclus, der zwei oder drei, vorzugsweise zwei Heteroatome, ausgewählt aus Stickstoff, Sauerstoff und Schwefel, kondensiert ist, nach Bindung an eine Matrix, vorzugsweise in der 2-Position, hinsichtlich einer selektiven Bindung an Albumin gescreent werden.

9. Verfahren nach Anspruch 8, **dadurch gekennzeichnet, dass** das Screening in wässrigen Lösungen bei einem pH, bei dem die Ligandstruktur einschließlich des Spacers, welcher an die Matrix bindet, positiv geladen ist, stattfindet.

## Revendications

1. Méthode pour enrichir/éliminer sélectivement une sérum-albumine à partir d'un mélange d'autres composés en mettant en contact ledit mélange avec un ligand (= X), ledit ligand
a) ayant une affinité pour la sérum-albumine et permettant sa liaison et
b) étant attaché via un espaceur (= B) à une matrice de base (= M') insoluble dans le milieu aqueux utilisé, la matrice avec le ligand attaché étant représentée par
M-B-X
M étant la matrice, B, l'espaceur et X le ligand d'affinité, à la condition que M puisse contenir d'autres groupes X liés via un espaceur,
**caractérisée en ce que** ledit ligand X a été choisi parmi des structures se liant à la sérum-albumine conformes à la formule 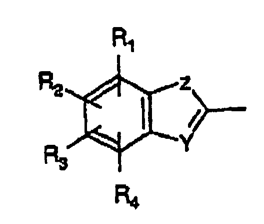 dans laquelle
a) la valence libre se lie à l'espaceur B ;
b) R₁₋₄ sont sélectionnés parmi l'hydrogène, des groupes électroattracteurs, tels que des halogènes et des groupes alkyle inférieur (C₁₋ ₁₀), dans lesquels au moins un de R₁₋₄ présente un groupe électroattracteur, de préférence choisi parmi des halogènes ;
c) z et y sont choisis parmi l'oxygène, le soufre ou l'azote, à condition que l'azote puisse porter une charge positive.

2. Méthode selon la revendication 1, **caractérisée en ce que** le contact entre le mélange et le milieu M-B-X est réalisé dans un milieu aqueux ayant un pH tel que le -B-X porte une charge positive.

3. Méthode selon l'une quelconque des revendications 1-2, **caractérisée en ce que** l'espaceur a un atome de soufre contigu à X.

4. Méthode selon l'une quelconque des revendications 1-3, **caractérisée en ce que** Z et Y sont des atomes d'azote, dont l'un se lie à un atome d'hydrogène et la structure du ligand étant chargée selon le pH.

5. Méthode de l'une quelconque des revendications 1-4, **caractérisée en ce que** ledit mélange provient d'un hôte dans lequel ladite sérum-albumine est la sérum-albumine humaine.

6. Méthode de l'une quelconque des revendications 1-5, **caractérisée en ce que** ledit ligand est attaché de manière covalente à ladite matrice.

7. Méthode de l'une quelconque des revendications 1-6, **caractérisée en ce que**, après l'étape d'adsorption ladite sérum-albumine est éluée dudit absorbant d'affinité et, si nécessaire, subit d'autres traitements.

8. Méthode de criblage pour des structures de ligand qui, lorsqu'elles sont attachées à une matrice d'affinité, se lient sélectivement à l'albumine, **caractérisée en ce que** des composés hydrosolubles qui possèdent un cycle benzénique substitué avec au moins un groupe électroattracteur fusionné à un hétérocycle à 5 chaînons contenant deux ou trois hétéro-atomes, de préférence deux, choisis parmi l'azote, l'oxygène et le soufre après avoir été attachés à une matrice, de préférence en position 2, sont criblés pour reconnaître ceux qui se lient sélectivement à l'albumine.

9. Méthode de la revendication 8, **caractérisée en ce que** le criblage a lieu dans des solutions aqueuses à un pH tel que la structure du ligand incluant l'espaceur se liant à la matrice est positivement chargée.
